# EUROPEAN PATENT SPECIFICATION

(11) **EP 2 411 086 B1**
(45) Date of publication and mention of the grant of the patent: **06.11.2013**
(21) Application number: 10704985.0
(22) Date of filing: 10.02.2010
(51) Int. Cl.: A61M 39/28

(54) **CLAMP FOR CLOSING FLEXIBLE TUBING BELONGING TO MEDICAL EQUIPMENT**
KLAMMER ZUM VERSCHLIESSEN EINES FLEXIBLEN SCHLAUCHS FÜR MEDIZINISCHE AUSRÜSTUNG
AGRAFE POUR FERMETURE D'UNE TUBULURE FLEXIBLE APPARTENANT A UN EQUIPEMENT MEDICAL

(30) Priority: 23.03.2009 IT MI20090447; 23.03.2009 IT MI20090450; 23.03.2009 IT MI20090451
(43) Date of publication of application: 01.02.2012
(73) Proprietor: Gambro Lundia AB, 220 10 Lund (SE)
(72) Inventor: LOMBARDO, Eugenio, I-32014 Ponte Nelle Alpi (IT); CALIMERI, Aldo, I-41037 Mirandola (IT)
(74) Representative: Ponzellini, Gianmarco
(86) International application number: PCT/IB2010/000254
(87) International publication number: WO 2010/109279

(56) References cited:
- WO-A-00/77428
- WO-A-2004/041343
- DE-U1- 29 902 927
- FR-A- 2 920 513
- GB-A- 2 448 374
- US-A- 3 822 052
- US-A- 4 589 626
- US-A- 5 318 546

## Description

The present invention relates to a clamp suitable for closing flexible tubing belonging to medical equipment. In particular, the present invention relates to elastic clamps.

Clamps of this type are used in equipment for extra corporal blood treatment, such as dialysis and/or filtration equipment (haemodialysis, haemofiltration, haemodiafiltration, ultrafiltration), and serve for closing the flexible tubing belonging to such equipment by deforming it, in order to prevent the passage of fluids (for example blood, infusion or replacement fluids) contained in the tubing itself.

The closing of the tubing made by the above clamps is required, for example, in emergency situations or when moving to a subsequent step of the treatment in progress. For example, the flexible tubing is temporarily closed for changing an empty bag of fluid. Clamps of known type are formed of a single plastic piece, which comprises a first arm, a second arm and a curved portion that elastically connects said two arms. A portion of the second arm opposite the curved portion extends towards a free end of the first arm and ends with a respective anchoring element. The free end of the first arm is provided with a respective anchoring element and with a manoeuvring portion. The two anchoring elements are set up for reciprocally engaging snap-wise when, acting with the fingers on the manoeuvring portion and on the second arm, the first arm is moved close to the second arm. The clamp further comprises two reciprocally facing projections and each one integral part of one of the two arms and has an opening obtained in the curved portion and an opening obtained in the portion of the second arm that extends towards the free end of the first arm. A flexible tubing is inserted in the two openings while the clamp is open, that is, while the two anchoring elements are not coupled, and it remains interposed between the two projections. By closing the clamp and coupling the anchoring elements, the projections reciprocally approach each other and squeeze the tubing up to fully closing the passage section of the same.

Clamps of the type described above are known for example from documents EP0995461 and EP0995462.

It is also known from document US 3822052 a clamp for one-hand manipulation in controlling the flow of fluid through flexible tubing, such as intravenous tubing systems, and formed from a moulded flat strip of plastic material configured to provide hinge areas for bending the strip to provide a base with an overlying lever arm between which the tubing is disposed, and a slotted locking arm upstanding from the base and having notches to receive a locking head on the lever arm for cooperation with the notches to hold abutment surfaces on the lever arm and base in position for clamping the tubing there between in completely or partially constricted position, and with permissible finger manipulation causing relative movement between the lever and locking arms to effect quick release of the interengaged head and notches, respectively. Document US 5318546 discloses a clamp for a plastic tube having a first arm with a first anchoring element positioned on a free end thereof and a second arm; a first connecting portion elastically connects the first arm with the second arm and has a first opening for the passage of a flexible tubing; a second connecting portion connects to the second arm opposite the first connecting portion, extends towards the free end of the first arm and has a second opening for the passage of the tube; a plurality of steps are positioned on the second connecting portion; a first projection with an inclined surface is integral to the first arm and faces the second arm; a second projection with an opposite inclined surface is integral to the second arm and faces the first projection; the flexible tubing passes through the first opening and the second opening and remains interposed between the first projection and the second projection; the first arm and the second arm are movable between an open position, wherein the first anchoring element and the second anchoring element are released and the projections are in a spaced position and inclined relationship, and a closed position, wherein the first anchoring element and the second anchoring element are coupled and the projections are in a close position and squeeze the tubing for completely closing a passage section of said tubing. Document WO 2004/1041343 discloses a clamp for closing flexible tubing belonging to medical equipment. It comprises a first arm having a first anchoring element positioned on a free end thereof and a second arm.

It also shows a first connecting portion connecting said first arm with said second arm and having a first opening for the passage of a flexible tubing; a second connecting portion connected to the second arm opposite the first connecting portion extends towards the free end of the first arm and has a second opening for the passage of the tubing.

A second anchoring element is positioned on the second connecting portion, a first projection is integral to the first arm and faces the second arm and a second projection is integral to the second arm and faces the first projection.

The flexible tubing passes through the first opening and the second opening remaining interposed between the first projection and the second projection, while the first arm and the second arm are movable between an open position, wherein the first anchoring element and the second anchoring element are released and the projections are in a spaced position, and a closed position wherein the first anchoring element and the second anchoring element are coupled and the projections are in a close position and squeeze the tubing.

The applicant has found that known clamps can be improved in many aspects from the functional and ergonomic point of view.

In particular, the Applicant has determined that known clamps offer poor usage practicality for the user. In fact, in the first place during the closing of the clamp on the tubing, besides reciprocally approaching each other, the two arms tend to slide relative to one another laterally, that is, to misalign relative to a symmetry plane of the clamp, making the reciprocal engagement of the anchoring elements difficult.

Moreover the opening of the known clamp is sometimes difficult to manage.

Moreover, again during the closure, known clamps tend to slip in the user's hand, making the closing operation complicated and slowing it, whereas it should be carried out in the least possible time.

The Applicant has further noted that known clamps mark the tubing also when they are open since the projections in any case press on the outside wall of the same. This is due to the fact that known clamps are made so that in their open and non-deformed configuration (without external constraints or forces applied to the clamp) the distance between the projections is in any case less than the tubing diameter, therefore when the tubing is inserted and spreads the arms, an elastic force closing the clamp is still present which releases on the piping and partially compresses it.

The present invention relates to a clamp according to claim 1.

There is also described a clamp for closing flexible tubing belonging to medical equipment, comprising: a first arm having a first anchoring element positioned on a free end thereof; a second arm; a first connecting portion elastically connecting said first arm with said second arm and having a first opening for the passage of a flexible tubing; a second connecting portion connected to the second arm opposite the first connecting portion, extending towards the free end of the first arm and having a second opening for the passage of said flexible tubing; a second anchoring element positioned on the second connecting portion; a first projection integral to the first arm and facing the second arm; a second projection integral to the second arm and facing the first projection; the flexible tubing passing through the first opening and the second opening remaining interposed between the first projection and the second projection; the first arm and the second arm being movable between an open position, wherein the first anchoring element and the second anchoring element are released and the projections are in a spaced position, and a closed position, wherein the first anchoring element and the second anchoring element are coupled and the projections are in a close position and squeeze the tubing for completely closing a passage section of said tubing; wherein the second arm has at least one recess shaped on a side edge thereof, for seating the user's forefinger, characterised in that the recess is arranged in the proximity of the second connecting portion.

There is also described a clamp for closing flexible tubing belonging to medical equipment, comprising: a first arm having a first anchoring element positioned on a free end thereof; a second arm; a first connecting portion elastically connecting said first arm with said second arm and having a first opening for the passage of a flexible tubing; a second connecting portion connected to the second arm opposite the first connecting portion, extending towards the free end of the first arm and having a second opening for the passage of said flexible tubing; a second anchoring element positioned on the second connecting portion; a first projection integral to the first arm and facing the second arm; a second projection integral to the second arm and facing the first projection; the flexible tubing passing through the first opening and the second opening) remaining interposed between the first projection and the second projection; the first arm and the second arm being movable between an open position, wherein the first anchoring element and the second anchoring element are released and the projections are in a spaced position, and a closed position, wherein the first anchoring element and the second anchoring element are coupled and the projections are in a close position and squeeze the tubing for completely closing a passage section of said tubing; characterised in that the first projection and the second projection have each a flat surface, said flat surfaces, when the first arm and the second arm are in the open position, being reciprocally parallel and abutted against the flexible tubing.

Within this scope, the technical task at the basis of the present invention is to provide a clamp suitable for closing flexible tubing belonging to medical equipment which should solve the drawbacks of the prior art mentioned above.

In particular, the object of the present invention is to provide a clamp capable of improving the functionality and making the use easier for the user.

A further object of the present invention is to propose a clamp which should cause the least possible damages to the flexible tubing whereon it is installed.

The above technical task and the specified objects are substantially achieved by a clamp suitable for closing flexible tubing belonging to medical equipment, comprising the technical features described in one or more of the annexed claims.

Further features and advantages of the present invention will appear more clearly from the exemplary and thus non limiting description of a preferred but non exclusive embodiment of a clamp suitable for closing hoses belonging to medical equipment, as illustrated in the annexed drawings, wherein:
- figure 1 shows a front perspective view of a clamp according to the invention;
- figure 2 shows a back perspective view of the clamp of figure 1;
- figure 3 shows a side elevation view of the clamp of figures 1 and 2;
- figure 4 shows a front elevation view of the clamp of figures 1 and 2;
- figure 5 shows a back elevation view of the clamp of figures 1 and 2;
- figures 6a and 6b show the clamp of figure 2 associated to a flexible tubing in respective operating configurations.

In the annexed figures, reference numeral 1 indicates a clamp suitable for closing flexible tubing belonging to medical equipment according to the present invention. Clamp 1 is optionally made in a single piece by moulding of plastic material, for example polypropylene, and is defined by a strip of material having a width larger than the thickness of the same.

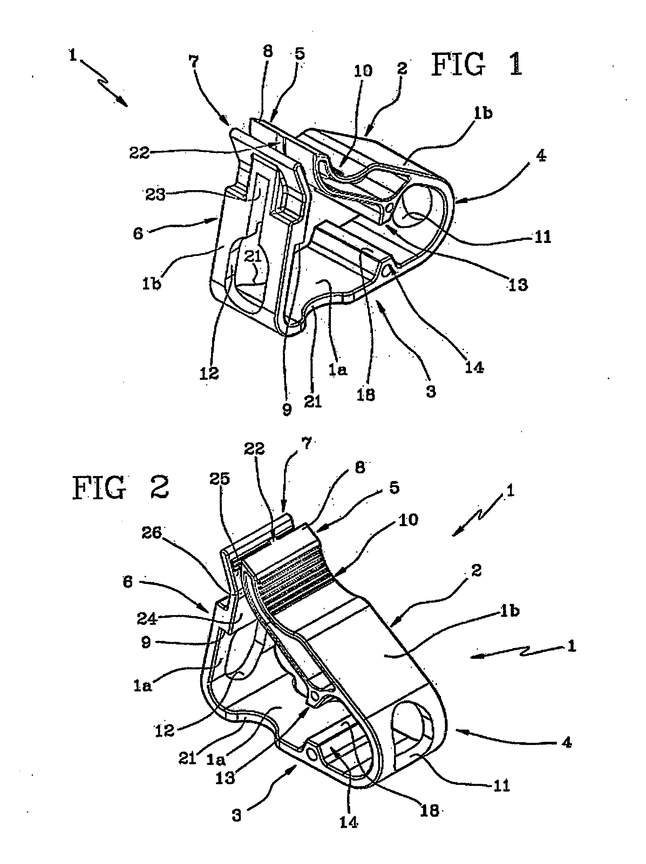

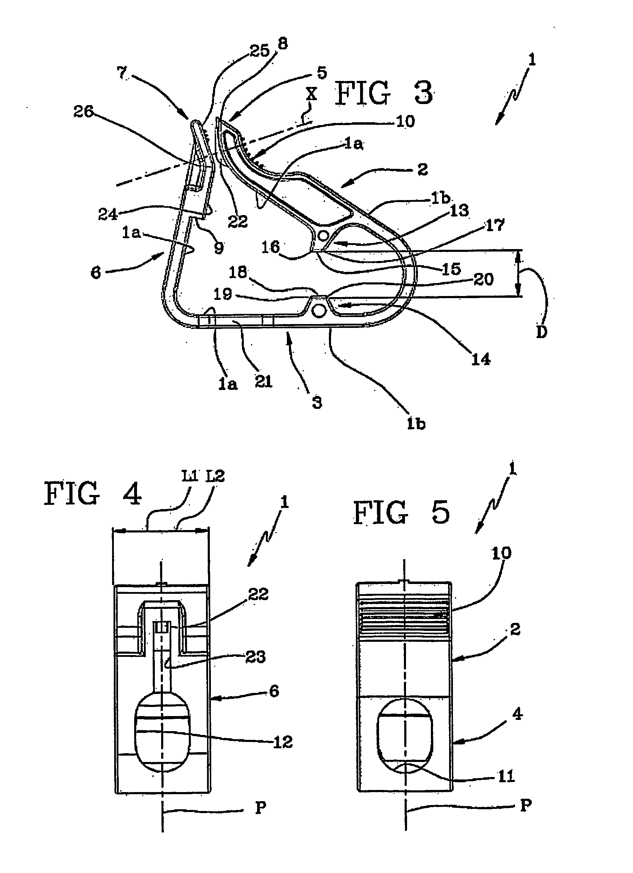

The above strip comprises a first arm 2 integrally connected to a second arm 3 by means of a first connecting portion 4. In particular, the first arm 2 has a free end 5 and an opposite end integral to the first connecting portion 4. The second arm 3 has an end integral to the first connecting portion 4 and an opposite end integral to a second connecting portion 6 that extends towards the free end 5 of the first arm 2 and ends with a respective free end 7. The first arm 2 has a rectilinear development, the second arm 3 has a rectilinear development, the first connecting portion 4 has a circle arc development, the second connecting portion 6 has a rectilinear development. In a non deformed condition (figures 1, 2 and 3), which as detailed hereinafter corresponds to an open position of clamp 1, the first arm 2 and the second arm 3 delimit an angle of about 45° with each other. Moreover, the second arm 3 and the second connecting portion 6 delimit an angle of about 80° with each other. In such non deformed condition, the free end 5 of the first arm 2 is further facing the free end 7 of the second connecting portion 6. In such condition, the strip is arranged ring-wise with its ends 5, 7 facing and has an internal peripheral surface 1a and an external peripheral surface 1b.

On the free end 5 of the first arm 2 there is a first anchoring element 8 which, in the embodiment shown, is an end portion of the same free end 5.

Moreover, on the second connecting portion 6 there is a second anchoring element 9 which, in the embodiment shown, is defined by a step positioned on the internal peripheral surface 1a of clamp 1 and is partially spaced from the free end 7 of the second portion 6. Step 9 delimits a stopping surface facing the second arm 3 and extends by the entire width of the second connecting portion 6.

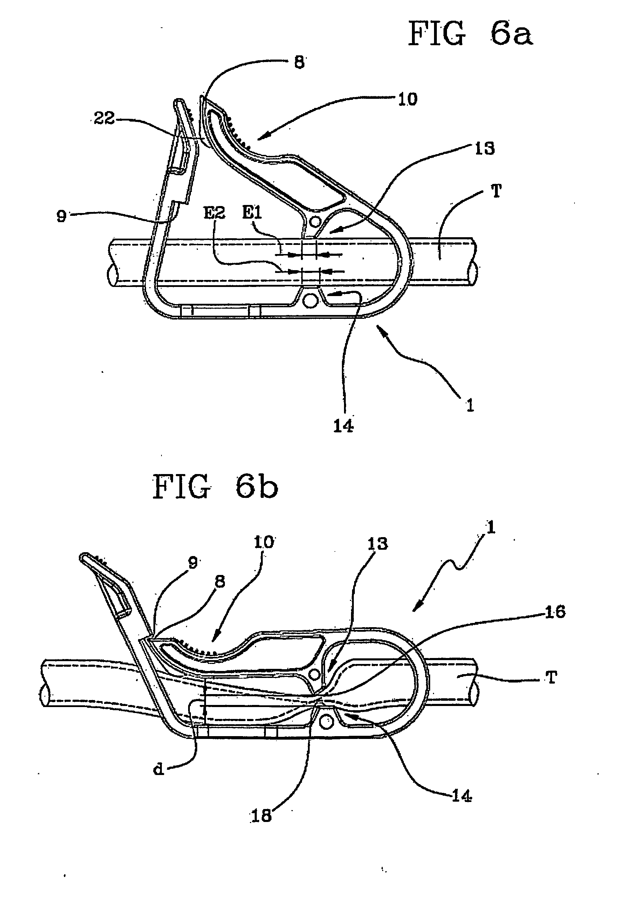

The first arm 3 has a seat 10 obtained on the external peripheral surface 1b and close to the free end 5 of the first arm 2. Seat 10 is a recessing concavity relative to the remaining external peripheral surface 1b of the first arm 2 and has projections or knurls suitable for increasing the friction with the finger placed thereon. By acting with the fingers of a hand, usually thumb and forefinger, on the second arm 3 and on seat 10 and moving the two arms 2, 3 close to one another, using the elastic deformation of the first portion 4 and of the second portion 6, the end portion 8 of the free end 5 of the first arm 2 is brought to step 9 and engaged snap-wise against the stopping surface (figures 6a and 6b). In such condition, clamp 1 is closed.

The first connecting portion 4 has a first opening 11 and the second connecting portion 6 has a second opening 12, which openings 11, 12 substantially lie aligned along a direction parallel to the second arm 3.

The first arm 2 further carries a first projection 13 that develops from its own internal peripheral surface 1a towards the second arm 3. The second arm 3 carries a second projection 14 that develops from its own internal peripheral surface 1a towards the first arm 2. The first and the second projection 13, 14 are reciprocally facing.

The first projection 13 extends by the entire width of the first arm 2 and in a section parallel to a longitudinal centre line plane "P" of clamp 1, it has a shape tapered as a trapezium starting from the base towards the end. At the end, the first projection 13 optionally has a flat surface 15 delimited by two edges 16, 17. A first edge 16 facing the second connecting portion 6 and a second edge 17 facing the first connecting portion 4. The flat surface 15 of the first projection 13 has a width "L₁" comprised between about 10mm and about 20mm, optionally equal to about 14mm. Moreover, the flat surface 15 of the first projection 13 has a longitudinal extension "E₁", measured in a plane parallel to the longitudinal centre line plane "P" of clamp 1, comprised between about 1mm and about 4mm. The first edge 16 has a bending radius "r" comprised between about 0.2mm and about 0.5mm, optionally equal to about 0.3mm.

The second projection 14 extends by the entire width of the second arm 3 and in a section parallel to a longitudinal centre line plane "P" of clamp 1, it has a shape tapered as a trapezium starting from the base towards the end. At the end, the second projection 14 has a flat surface 18 delimited by two edges 19, 20. The flat surface 18 of the second projection 14 has a width "L2" comprised between about 10mm and about 20mm, optionally equal to about 14mm. Moreover, the flat surface 18 of the second projection 14 has a longitudinal extension "E₂", measured in a plane parallel to the longitudinal centre line plane "P" of clamp 1, comprised between about 1mm and about 4mm.

While clamp 1 is open, with the anchoring elements 8, 9 released and projections 13, 14 spaced from each other, a flexible tubing "T" is inserted through the first and the second opening 11, 12 and thus a portion of the same tubing "T" remains inside clamp 1 and interposed between the two projections 13, 14 (figure 6a).

In such open position, the flat surface 15 of the first projection 13 and the flat surface 18 of the second projection 14 are parallel and abutted against opposite portions of the flexible tubing "T".

Closing clamp 1, according to the description above, projections 13, 14 are moved close to each other and also rotated relative to each other. Such approach causes the squeezing of tubing "T", up to closing the inside passage section of tubing "T" itself (figure 6b). In the closed position of clamp 1, when the first and the second anchoring element 8, 9 are reciprocally coupled, the flat surface 18 of the second projection 14 still contacts tubing "T" whereas the first projection 13 presses on tubing "T" with its first edge 16, which therefore faces said flat surface 18.

In the open position, the first projection 13 and the second projection 14 are spaced by a distance "D" comprised between about 6mm and about 7mm, optionally equal to about 6.6mm.

In the closed position, the first projection 13 and the second projection 14 are spaced by a minimum distance "d" comprised between about 0.2mm and about 0.7mm, optionally equal to about 0.5mm.

Optionally, the second arm 3 has, at one or both its side edges, a recess 21 arranged in the proximity of the second connecting portion 6. Such recesses 21, which in the embodiment illustrated are symmetrical relative to the longitudinal centre line plane "P", seat the user's forefinger while he/she manoeuvres clamp 1.

Clamp 1 comprises also a first guiding element 22 associated to the first arm 2 and a second guiding element 23 associated to the second arm 3.

The guiding elements 22, 23 engage reciprocally during the movement between the open position and the closed position of clamp 1 and prevent the side movement of the first arm 2 and of the second arm 3 relative to each other and relative to the longitudinal centre line plane of clamp 1 during the movement between the open and the closed positions.

It is to be noted that in the closed position of the first arm 2 and the second arm 3, the first guiding element 22 and the second guiding element 23 are disengaged (see fig. 1). The above means that, in the closed position of the first arm 2 and the second arm 3, the first guiding element 22 and the second guiding element 23 allow the side movement of the first arm 2 and of the second arm 3 relative to a longitudinal centre line plane P of the clamp 1.

This lateral slide movement can help in easy and quick disengagement of the two arms.

Optionally also in the open position of the first arm 2 and the second arm 3, the first guiding element 22 and the second guiding element 23 are disengaged.

In other words in the open position the first guiding element 22 and the second guiding element 23 allow the side movement of the first arm 2 and of the second arm 3 relative to a longitudinal centre line plane P of the clamp 1.

In the illustrated embodiment, the first guiding element 22 is a tab positioned at the free end 5 of the first arm 2 on the internal surface 1a and in a zone opposite seat 10 for the thumb. Tab 22 extends parallel to the longitudinal centre line plane "P" and has an arched edge.

The second guiding element 23 is a groove obtained in the second connecting portion 6 at the free end 7. More in detail, groove 23 develops along the longitudinal centre line plane "P", optionally as continuation of the second opening 12. Groove 23 has such a width as to seat tab 22 but prevent or at least limit side movements of the latter, or movements orthogonal to the longitudinal centre line plane "P" of clamp 1 at least during the engaging movement.

According to a not shown embodiment, the elements are inverted, that is, the tab is positioned at the free end 7 of the second connecting portion 6 and groove 23 is obtained at the free end 5 of the first arm 2. Optionally, groove 23 is interposed between the free end 7 of the second connecting portion 6 and step 9. In fact, step 9 is positioned in the proximity of an end of groove 23 close to the second opening 12. In the embodiment shown, step 9 is positioned at the zone of junction of groove 23 into the second opening 12.

Step 9 separates the internal peripheral surface 1a belonging to the second connecting portion 6 into two surfaces lying on staggered planes. The internal surface 1a interposed between step 9 and the free end 7 is in turn divided into a first surface 24 parallel to the remaining internal surface 1a but closer to the first arm 2 and a second internal surface 25 inclined relative to the first surface 24. The second inclined surface 25 develops towards the external peripheral surface 1b of the same second connecting portion 6, that is, away from the free end 5 of the first arm 2, and with the first surface 24 it forms an edge 26 and an angle of about 160°.

Groove 23 develops in the second connecting portion 6 at the first surface 24 and partially, in the second internal inclined surface 25, without reaching the free end 7.

Moreover, the positioning zone of tab 22 located on the first arm 2 has a concave and arched shape that in the open position of clamp 1, faces the inclined surface 25 mentioned above.

In use, in the open position of clamp 1, visible in figures 1-5, at least a part of the support seat 10, tab 22 and groove 23 are reciprocally aligned along an initial movement direction "X" of arms 2 and 3.

In such position, while the flexible tubing "T" is inserted in clamp 1 and is open, since projections 13, 14 are spaced from each other, tab 22 faces the inclined surface 25 and external to groove 23 (figure 6a). Projections 13, 14 slightly compress tubing "T" so as to hold clamp 1 on tubing "T" itself.

When the user pushes the free end 5 of the first arm 2 towards the second connecting portion 6, the concave and arched positioning zone of tab 22 rests against the inclined surface 25 and tab itself 22 enters into groove 23. The concave and arched zone slides on the inclined surface 25 and then on the first surface 24 and tab 22 slides into groove 23 until the free end 5 of the first arm 2 reaches step 9, where it engages snap-wise against the stopping surface (figure 6b, closed position of clamp 1), using the elasticity of the second connecting portion 6. Tab 22 in the meantime has come out of groove 23.

To open clamp 1, it is sufficient to manually move the free end 7 of the second connecting portion 6 away from the first arm 2, optionally provided with rises or knurls. The first arm 2, thanks to the elastic return of the first connecting portion 4 and to the elastic return of the flexible tubing "T" interposed between projections 13, 14, returns to the open position.

Thanks to the guiding elements (tab 22 and groove 23), the reciprocal side movement of the closing arms is prevented, or in any case strongly limited. As a consequence, the closing movement of the clamp is more accurate and easy to be carried out by the user.

The presence of the guiding elements further allows making the clamp so that, when the clamp is open in the rest position, that is, is not subject to any constraint or external force (the flexible tubing is not present which in known clamps, keeps the projections spread), the distance between the projections is much larger than that of the prior art clamps and substantially equal, a little smaller, than the outside diameter of tubing "T". In this situation when the tubing is inserted, the elastic closing of the clamp open on the tubing itself exerts a much lighter force than that exerted by the known clamps and marks the tubing less.

The movement of the first arm 2 relative to the second arm 3 for achieving the lock certainly is greater than in known type clamps but such movement is advantageously guided by the guiding elements.

Also the shape of projections 13 and 14 contributes to decreasing the impression of the same on the tubing when the clamp is open, since the flat surfaces 15, 18 allow increasing the contact area with the tubing itself.

Seat 10 for the thumb together with recesses 21 for the forefinger ensure a safer grip for the user and thus increase the usage practicality.

## Claims

1. Clamp for closing flexible tubing belonging to medical equipment, comprising:
a first arm (2) having a first anchoring element (8) positioned on a free end thereof (5);
a second arm (3);
a first connecting portion (4) elastically connecting said first arm (2) with said second arm (3) and having a first opening (11) for the passage of a flexible tubing (T);
a second connecting portion (6) connected to the second arm (3) opposite the first connecting portion (4),
extending towards the free end (5) of the first arm (2) and having a second opening (12) for the passage of said flexible tubing (T);
a second anchoring element (9) positioned on the second connecting portion (6);
a first projection (13) integral to the first arm (2) and facing the second arm (3);
a second projection (14) integral to the second arm (3) and facing the first projection (13); the flexible tubing (T) passing through the first opening (11) and
the second opening (12) remaining interposed between the first projection (13) and the second projection (14);
the first arm (2) and the second arm (3) being movable between an open position, wherein the first anchoring element (8) and the second anchoring element (9) are released and the projections (13, 14) are in a spaced position, and a closed position, wherein the first anchoring element (8) and the second anchoring element (9) are coupled and the projections (13, 14) are in a close position and squeeze the tubing (T) for completely closing a passage section of said tubing (T);
a first guiding element (22) associated to the first arm (2) and
a second guiding element (23) associated to the second arm (3); said guiding elements (22, 23) reciprocally engaging during the movement between the open position and the closed position and preventing the side movement of the first arm (2) and of the second arm (3) relative to a longitudinal centre line plane (P) of the clamp (1), **characterized in that** in the closed position of the first arm (2) and the second arm (3) the first guiding element (22) and the second guiding element (23) are disengaged allowing the side movement of the first arm (2) and of the second arm (3) relative to a longitudinal centre line plane (P) of the clamp (1).

2. Clamp according to claim 1, wherein the first guiding element (22) is a tab positioned at the free end (5) of the first arm (2) and the second guiding element (23) is a groove obtained at a free end (7) of the second connecting portion (6), or alternatively
wherein the first guiding element (22) is a groove obtained at the free end (5) of the first arm (2) and the second guiding element (23) is a tab positioned at a free end (7) of the second connecting portion (6).

3. Clamp according to claim 1 or 2, wherein the first arm (2) has a support seat (10) for a finger of the user obtained at the free end (5) of said first arm (2) and wherein the first guiding element (22) is positioned on a surface of the first arm (2) close to the free end (5) and opposite said support seat (10),
the support seat (10) being a recessing concavity relative to a remaining external surface (1b) of the first arm (2).

4. Clamp according to the previous claim, wherein at least in the open position of the clamp (1), at least a part of the support seat (10), the first guiding element (22) and the second guiding element (23) are reciprocally aligned along a direction (X) of initial movement of said arms (2, 3).

5. Clamp according to any one of the previous claims, wherein the second guiding element (23) is interposed between the free end (7) of the second connecting portion (6) and the second anchoring element (9).

6. Clamp according to any one of the previous claims, wherein the first anchoring element (8) is an end portion of said free end (5) and the second anchoring element (9) is a step.

7. Clamp according to any one of the previous claims, wherein in the open position of the first arm (2) and the second arm (3), the first guiding element (22) and the second guiding element (23) the first guiding element (22) and the second guiding element (23) are disengaged.

8. Clamp according to any one of the previous claims, wherein in the open position of the first arm (2) and the second arm (3), the first guiding element (22) and the second guiding element (23) allow the side movement of the first arm (2) and of the second arm (3) relative to a longitudinal centre line plane (P) of the clamp (1).

9. Clamp according to any one of the previous claims, wherein the second arm (3) has at least one recess (21) shaped on a side edge thereof, for seating the user's forefinger, the recess (21) being arranged in the proximity of the second connecting portion (6).

10. Clamp according to claim 9, wherein the second arm (3) has two recesses (21), each shaped on a respective side edge, in particular the two recesses (21) being arranged in the proximity of the second connecting portion (6) and optionally being
symmetrical relative to a longitudinal centre line plane (P) of the clamp (1).

11. Clamp according to any one of the previous claims, wherein the first projection (13) and the second projection (14) have each a flat surface (15, 18), said flat surfaces (15, 18), when the first arm (2) and the second arm (3) are in the open position, being reciprocally parallel and abutted against the flexible tubing (T).

12. Clamp according to claim 11, wherein the first projection (13) has an edge (16) and wherein said edge (16), when the first arm (2) and the second arm (3) are in the closed position, faces the flat surface (18) of the second projection (14) and acts against the flexible tubing (T).

13. Clamp according to claim 11 or 12, wherein the flat surface (15) of the first projection (13) has a longitudinal extension (E₁) comprised between about 1 mm and about 3 mm and
wherein the flat surface (18) of the second projection (14) has a longitudinal extension (E₂) comprised between about 1 mm and about 4 mm.

14. Clamp according to claim 11, 12 or 13, wherein the flat surface (15) of the first projection (13) has a width (L₁) comprised between about 10 mm and about 20 mm, optionally equal to about 14 mm and
wherein the flat surface (18) of the second projection (14) has a width (L2) comprised between about 10 mm and about 20 mm, optionally equal to about 14 mm.

15. Clamp according to claim 12, wherein the edge (16) of the first projection (13) has a bending radius comprised between about 0.2 mm and about 0.5 mm, optionally equal to about 0.3 mm and
wherein when the first arm (2) and the second arm (3) are in their closed position, the minimum distance (d) between the two projections (13, 14) is comprised between about 0.2 mm and about 0.7 mm, optionally equal to about 0.5 mm.

## Patentansprüche

1. Klemme zum Schließen flexibler Schläuche medizinischer Ausrüstung, umfassend:
einen ersten Arm (2) mit einem ersten Ankerelement (8), das an einem freien Ende (5) dessen angeordnet ist;
einen zweiten Arm (3);
einen ersten Verbindungsteil (4), der den ersten Arm (2) mit dem zweiten Arm (3) elastisch verbindet und eine erste Öffnung (11) für den Durchgang eines flexiblen Schlauches (T) aufweist;
einen zweiten Verbindungsteil (6), der mit dem zweiten Arm (3) gegenüberliegend des ersten Verbindungsteils (4) verbunden ist und sich zum freien Ende (5) des ersten Arms (2) erstreckt und eine zweite Öffnung (12) für den Durchgang des Schlauches (T) aufweist;
ein zweites Ankerelement (9), das am zweiten Verbindungsteil (6) angeordnet ist;
einen ersten Vorsprung (13), der mit dem ersten Arm (2) einstückig ausgebildet und dem zweiten Arm (3) zugewandt ist;
einen zweiten Vorsprung (14), der mit dem zweiten Arm (3) einstückig ausgebildet und dem ersten Vorsprung (13) zugewandt ist; wobei der flexible Schlauch (T), der durch die erste Öffnung (11) und die zweite Öffnung (12) durch geht, zwischen dem ersten Vorsprung (13) und dem zweiten Vorsprung (14) bleibt;
wobei der erste Arm (2) und der zweite Arm (3) zwischen einer offenen Stellung, in der das erste Ankerelement (8) und das zweite Ankerelement (9) freigegeben werden und die Vorsprünge (13, 14) in einer beabstandeten Stellung sind, und einer geschlossenen Stellung, in der das erste Ankerelement (8) und das zweite Ankerelement (9) gekoppelt sind und die Vorsprünge (13, 14) in einer Schließstellung sind und den Schlauch (T) zum vollständigen Schließen eines Durchgangsabschnitts des Schlauches (T) zusammendrücken, bewegbar sind;
ein erstes Führungselement (22), das dem ersten Arm (2) angegliedert ist, und
ein zweites Führungselement (23), das dem zweiten Arm (3) angegliedert ist; wobei die Führungselemente (22, 23) bei der Bewegung zwischen der offenen Stellung und der geschlossenen Stellung gegenseitig ineinander eingreifen und die seitliche Bewegung des ersten Arms (2) und des zweiten Arms (3) bezüglich einer Längsmittenlinienebene (P) der Klemme (1) verhindern, **dadurch gekennzeichnet, daß** in der geschlossenen Stellung des ersten Arms (2) und des zweiten Arms (3) das erste Führungselement (22) und das zweite Führungselement (23) entkoppelt sind und die seitliche Bewegung des ersten Arms (2) und
des zweiten Arms (3) bezüglich einer Längsmittenlinienebene (P) der Klemme (1) ermöglichen.

2. Klemme nach Anspruch 1, wobei das erste Führungselement (22) eine Zunge ist, die am freien Ende (5) des ersten Arms (2) angeordnet ist, und das zweite Führungselement (23) eine Nut ist, die an einem freien Ende (7) des zweiten Verbindungsteils (6) erhalten ist, oder alternativ wobei das erste Führungselement (22) eine Nut ist, die am freien Ende (5) des ersten Arms (2) erhalten ist, und das zweite Führungselement (23) eine Zunge ist, die an einem freien Ende (7) des zweiten Verbindungsteils (6) angeordnet ist.

3. Klemme nach Anspruch 1 oder 2, wobei der erste Arm (2) einen Stützsitz (10) für einen Finger des Benutzers, der am freien Ende (5) des ersten Arms (2) erhalten ist, aufweist, und wobei das erste Führungselement (22) auf einer Fläche des ersten Arms (2) neben dem freien Ende (5) und gegenüber dem Stützsitz (10) angeordnet ist, wobei der Stützsitz (10) eine vertiefte Wölbung bezüglich einer verbleibenden Außenfläche (1b) des ersten Arms (2) ist.

4. Klemme nach dem vorhergehenden Anspruch, wobei mindestens in der offenen Stellung der Klemme (1) mindestens ein Teil des Stützsitzes (10), das erste Führungselement (22) und das zweite Führungselement (23) entlang einer Richtung (X) einer Anfangsbewegung der Arme (2, 3) miteinander fluchtend sind.

5. Klemme nach einem der vorhergehenden Ansprüche, wobei das zweite Führungselement (23) zwischen dem freien Ende (7) des zweiten Verbindungsteils (6) und dem zweiten Ankerelement (9) angeordnet ist.

6. Klemme nach einem der vorhergehenden Ansprüche, wobei das erste Ankerelement (8) ein Endabschnitt des freien Endes (5) und das zweite Ankerelement (9) eine Stufe ist.

7. Klemme nach einem der vorhergehenden Ansprüche, wobei in der offenen Stellung des ersten Arms (2) und des zweiten Arms (3) das erste Führungselement (22) und das zweite Führungselement (23) das erste Führungselement (22) und das zweite Führungselement (23) entkoppelt sind.

8. Klemme nach einem der vorhergehenden Ansprüche, wobei in der offenen Stellung des ersten Arms (2) und des zweiten Arms (3) das erste Führungselement (22) und das zweite Führungselement (23) die seitliche Bewegung des ersten Arms (2) und des zweiten Arms (3) bezüglich einer Längsmittenlinienebene (P) der Klemme (1) ermöglichen.

9. Klemme nach einem der vorhergehenden Ansprüche, wobei der zweite Arm (3) mindestens eine Vertiefung (21), die an einem Seitenrand dessen geformt ist, aufweist, zur Aufnahme des Zeigefingers des Benutzers, wobei die Vertiefung (21) in der Nähe des zweiten Verbindungsteils (6) angeordnet ist.

10. Klemme nach Anspruch 9, wobei der zweite Arm (3) zwei Vertiefungen (21) aufweist, wobei jede davon an einem entsprechenden Seitenrand geformt ist, wobei insbesondere die beiden Vertiefungen (21) in der Nähe des zweiten Verbindungsteils (6) angeordnet sind und optional symmetrisch bezüglich einer Längsmittenlinienebene (P) der Klemme (1) sind.

11. Klemme nach einem der vorhergehenden Ansprüche, wobei der erste Vorsprung (13) und der zweite Vorsprung (14) jeweils eine flache Oberfläche (15, 18) besitzen, wobei die flachen Oberflächen (15, 18), wenn der erste Arm (2) und der zweite Arm (3) in der offenen Stellung sind, zueinander parallel sind und am Schlauch (T) anliegen.

12. Klemme nach Anspruch 11, wobei der erste Vorsprung (13) einen Rand (16) aufweist und wobei der Rand (16), wenn der erste Arm (2) und der zweite Arm (3) in der geschlossenen Stellung sind, der flachen Oberfläche (18) des zweiten Vorsprungs (14) zugewandt ist und auf den Schlauch (T) wirkt.

13. Klemme nach Anspruch 11 oder 12, wobei die flache Oberfläche (15) des ersten Vorsprungs (13) eine Längsausdehnung (E₁) von etwa 1 mm bis etwa 3 mm aufweist und wobei die flache Oberfläche (18) des zweiten Vorsprungs (14) eine Längsausdehnung (E₂) von etwa 1 mm bis etwa 4 mm aufweist.

14. Klemme nach Anspruch 11, 12 oder 13, wobei die flache Oberfläche (15) des ersten Vorsprungs (13) eine Breite (L₁) von etwa 10 mm bis etwa 20 mm, optional von etwa 14 mm, aufweist und wobei die flache Oberfläche (18) des zweiten Vorsprungs (14) eine Breite (L2) von etwa 10 mm bis etwa 20 mm, optional von etwa 14 mm, aufweist.

15. Klemme nach Anspruch 12, wobei der Rand (16) des ersten Vorsprungs (13) einen Biegeradius von etwa 0,2 bis etwa 0,5 mm, optional von etwa 0,3 mm, aufweist und wobei, wenn der erste Arm (2) und der zweite Arm (3) in deren geschlossenen Stellung sind, der Minimalabstand (d) zwischen den beiden Vorsprüngen (13, 14) etwa 0,2 mm bis etwa 0,7 mm, optional etwa 0,5 mm, beträgt.

## Revendications

1. Etau pour fermer un tuyau flexible faisant partie d'un appareil médical, comprenant:
un premier bras (2) ayant un premier élément d'ancrage (8) placé sur un extrémité libre (5) de celui-ci;
un deuxième bras (3);
une première portion de liaison (4) reliant élastiquement ledit premier bras (2) audit deuxième bras (3) et ayant une première ouverture (11) pour le passage d'un tuyau flexible (T);
une deuxième portion de liaison (6) reliée au deuxième bras (3) en face de ladite première portion de liaison (4), s'étendant vers l'extrémité libre (5) du premier bras (2) et ayant une deuxième ouverture (12) pour le passage d'un tuyau flexible (T);
un deuxième élément d'ancrage (9) placé sur la deuxième portion de liaison (6);
une première saillie (13) solidaire au premier bras (2) et en face du deuxième bras (3);
une deuxième saillie (14) solidaire au deuxième bras (3) et en face de la première saillie (13); le tuyau flexible (T) passant à travers la première ouverture (11) et la deuxième ouverture (12) restant interposée entre la première saillie (13) et la deuxième saillie (14);
le premier bras (2) et le deuxième bras (3) étant mobiles entre une position ouverte, où le premier élément d'ancrage (8) et le deuxième élément d'ancrage (9) sont dégagés et les saillies (13, 14) sont dans une position espacée, et une position fermée, où le premier élément d'ancrage (8) et le deuxième élément d'ancrage (9) sont accouplés et les saillies (13, 14) sont dans une position de fermeture et pressent le tuyau (T) pour fermer complètement une section de passage dudit tuyau (T);
un premier élément de guide (22) associé au premier bras (2), et
un deuxième élément de guide (23) associé au deuxième bras (3); lesdits éléments de guide (22, 23) s'engageant réciproquement pendant le mouvement entre la position ouverte et la position fermée et empêchant le mouvement latéral du premier bras (2) et du deuxième bras (3) par rapport à un plan de ligne moyenne longitudinale (P) de l'étau (1), **caractérisé en ce que** dans la position fermée du premier bras (2) et du deuxième bras (3) le premier élément de guide (22) et le deuxième élément de guide (23) sont dégagés et permettent le mouvement latéral du premier bras (2) et du deuxième bras (3) par rapport à un plan de ligne moyenne longitudinale (P) de l'étau (1).

2. Etau selon la revendication 1, où le premier élément de guide (22) est une patte placée à l'extrémité libre (5) du premier bras (2) et le deuxième élément de guide (23) est une rainure obtenue à une extrémité libre (7) de la deuxième portion de liaison (6), ou alternativement où le premier élément de guide (22) est une rainure obtenue à l'extrémité libre (5) du premier bras (2) et le deuxième élément de guide (23) est une patte placée à une extrémité libre (7) de la deuxième portion de liaison (6).

3. Etau selon la revendication 1 ou 2, où le premier bras (2) a un siège de support (10) pour un doigt de l'utilisateur obtenu à l'extrémité libre (5) dudit premier bras (2), et où le premier élément de guide (22) est placé sur une surface du premier bras (2) proche de l'extrémité libre (5) et en face dudit siège de support (10), le siège de support (10) étant une concavité rentrante par rapport à une surface extérieure (1 b) restante du premier bras (2).

4. Etau selon la revendication précédente, où au moins dans la position ouverte de l'étau (1) au moins une partie du siège de support (10), le premier élément de guide (22) et le deuxième élément de guide (23) sont réciproquement en ligne le long d'une direction (X) de mouvement initial desdits bras (2, 3).

5. Etau selon une quelconque des revendications précédentes, où le deuxième élément de guide (23) est placé entre l'extrémité libre (7) de la deuxième portion de liaison (6) et le deuxième élément d'ancrage (9).

6. Etau selon une quelconque des revendications précédentes, où le premier élément d'ancrage (8) est une portion d'extrémité de ladite extrémité libre (5) et le deuxième élément d'ancrage (9) est une marche.

7. Etau selon une quelconque des revendications précédentes, où dans la position ouverte du premier bras (2) et du deuxième bras (3), le premier élément de guide (22) et le deuxième élément de guide (23) le premier élément de guide (22) et le deuxième élément de guide (23) sont dégagés.

8. Etau selon une quelconque des revendications précédentes, où dans la position ouverte du premier bras (2) et du deuxième bras (3), le premier élément de guide (22) et le deuxième élément de guide (23) permettent le mouvement latéral du premier bas (2) et du deuxième bras (3) par rapport à un plan de ligne moyenne longitudinale (P) de l'étau (1).

9. Etau selon une quelconque des revendications précédentes, où le deuxième bras (3) a au moins une niche (21) formée sur un bord latéral de celui-ci, pour loger l'index de l'utilisateur, la niche (21) étant placée proche de la deuxième portion de liaison (6).

10. Etau selon la revendication 9, où le deuxième bras (3) a deux niches (21), chacune étant formée sur un bord latéral respectif, en particulier les deux niches (21) étant placées près de la deuxième portion de liaison (6) et de façon optionnelle étant symétriques par rapport à un plan de ligne moyenne longitudinale (P) de l'étau (1).

11. Etau selon une quelconque des revendications précédentes, où la première saillie (13) et la deuxième saillie (14) ont chacune une surface plate (15, 18), lesdites surfaces plates (15, 18), lorsque le premier bras (2) et le deuxième bras (3) sont dans la position ouverte, étant réciproquement parallèles et s'appuyant contre le tuyau flexible (T).

12. Etau selon la revendication 11, où la première saillie (13) a un bord (16) et où ledit bord (16), lorsque le premier bras (2) et le deuxième bras (3) sont dans la position fermée, est en face de la surface plate (18) de la deuxième saillie (14) et agit contre le tuyau flexible (T).

13. Etau selon la revendication 11 ou 12, où la surface plate (15) de la première saillie (13) a une extension longitudinale (E₁) allant d'environ 1 mm à environ 3 mm, et où la surface plate (18) de la deuxième saillie (14) a une extension longitudinale (E₂) allant d'environ 1 mm à environ 4 mm.

14. Etau selon la revendication 11, 12 ou 13, où la surface plate (15) de la première saillie (13) a une largeur (L₁) allant d'environ 10 mm à environ 20 mm, de façon optionnelle d'environ 14 mm, et où la surface plate (18) de la deuxième saillie (14) a une largeur (L2) allant d'environ 10 mm à environ 20 mm, de façon optionnelle d'environ 14 mm.

15. Etau selon la revendication 12, où le bord (16) de la première saillie (13) a un rayon de courbure allant d'environ 0,2 mm à environ 0,5 mm, de façon optionnelle d'environ 0,3 mm, et où lorsque le premier bras (2) et le deuxième bras (3) sont dans leur position fermée, la distance minimum (d) entre les deux saillies (13, 14) est comprise entre environ 0,2 mm et environ 0,7 mm, de façon optionnelle est d'environ 0,5 mm.
